# EUROPEAN PATENT APPLICATION

(11) **EP 4 847 000 A1**
(43) Date of publication of application: **02.12.2026**
(21) Application number: 24920039.5
(22) Date of filing: 24.01.2024
(51) Int. Cl.: A61K 6/58

(54) **SOLUTION FOR REGENERATING PASSIVATION OF DENTAL IMPLANTS AND REDUCING BACTERIAL ACTIVITY, AND USES OF SAID SOLUTION**

(71) Applicant: SOADCO, S.L., Escaldes-Engordany AD700 (AD)
(72) Inventor: MARTÍNEZ LÓPEZ, Jorge, AD200 ENCAMP (AD); GIL MUR, Javier, 08022 Barcelona (ES); BRIZUELA VELASCO, Aritza, 48007 Bilbao (Vizcaya) (ES)
(74) Representative: Canela Giménez, Arturo
(86) International application number: PCT/ES2024/070040
(87) International publication number: WO 2025/158088

(57) **Abstract**

A solution for regenerating the passivation layer on dental implants and reducing bacterial activity, comprising between 0.2 and 1.0 grams of collagen and between 5 and 50 grams of divalent magnesium or calcium ion salts for every litre of a 10-50% (v/v) citric acid solution. The invention comprises the use of the solution as a mouthwash, an agent for enhancing hydrophilicity and osseointegration, and an agent for reducing bacterial activity in dental implants.

## Description

### Technical field

The present invention relates to a solution for regenerating the passivation layer on dental implants, including abutments made of osteogenic materials, and reducing bacterial activity. Such solution is applicable in the field of dental health, specifically in relation to dental implants and components such as crowns and abutments used in dental implantology.

### Prior art

Nowadays, dental implant placement is the most widely used treatment for the replacement of patients' teeth. These dental implants have allowed patients to restore their appearance, chewing ability and speech.

Although the success rate of this treatment is high, complications may arise in some cases due to adhesion of bacterial colonies or poor osseointegration.

To prevent the failure of dental implants, the physico-chemical properties and surface topography must be taken into account, as these are crucial to increase osseointegration rate and to reduce bacterial colonisation on the surface.

Therefore, the technical challenge is to enhance the osseointegration and antibacterial properties of implants, abutments and crowns in order to increase the success rate of implant placement.

### Description of the invention

To solve the technical problem described, a liquid solution has been developed to produce a coating with antibacterial and osseointegrative properties on dental implants, abutments and crowns by immersing them in this solution for a given period of time.

According to the invention, such solution comprises collagen, a citric acid solution and salts of divalent ions such as magnesium or calcium in the following proportions: between 0.2 and 1.0 grams of collagen and between 5 and 50 grams of divalent magnesium or calcium ion salts for every litre of 10-50% (v/v) citric acid solution.

The ability to enhance osseointegration and antibacterial properties, whilst also increasing the success rate of implant placements, provides a solution for machined surfaces following an implantoplasty.

In order to develop the invention, studies were performed to determine the immersion time of the implant/crown in the solution based on the collagen concentration and contact angle measurements on the surface. Immersion times of less than 10 minutes were used, taking into account the clinical application of the solution.

The cell response (cytotoxicity assessment) of samples was studied at the previously determined optimal immersion time by observing cell adhesion using osteoblasts (SaOS-2).

Finally, the antibacterial properties of samples at the optimal time were analysed by monitoring the growth of bacterial strain *Streptococcus gordonii*.

This invention includes the use of the aforementioned solution as: a mouthwash for dental health applied on dental implants and their components; an agent to improve the hydrophilicity of dental implant surfaces; an agent to improve osseointegration of dental implants; and an agent to reduce bacterial activity within the surface of dental implants.

### Brief description of the drawings

To further illustrate the description provided herein, this specification is accompanied by a set of drawings in which, by way of illustration and without limitation, graphs show the results of experimental methods conducted using the solution of the invention, specifically:
- Figure 1 shows a graph with the results of contact angle measurements performed on the surfaces of discs made from different materials. Measurements were conducted on control samples (Ctrl) and samples treated with the solution of the invention for 2 and 10 minutes.
- Figure 2 shows a graph with the cytocompatibility results for different materials subjected to different treatments.
- Figure 3 shows a graph with the results for bacterial adhesion using the Alamar Blue assay.

### Detailed description of the embodiments of the invention

The materials and experimental methods are described below.

Discs made of titanium (grade 4), zirconium, composite, and cobalt-chromium were immersed in four different solutions. After immersion for 2 and 10 minutes, samples were analysed using scanning electron microscopy (SEM) and the contact angle (CA) was measured to establish optimal treatment time and to determine surface wettability.

The solutions being evaluated are shown in the table below:

| **TREATMENT** | **CONDITIONS** |
|---|---|
| 1 | Control without disc (glass plate) |
| 2 | Control with disc (grade 4 titanium, zirconium, composite, and cobalt-chromium) |
| 3 | 25% citric acid |
| 4 | 25% citric acid + collagen 250 |
| 5 | 25% citric acid + collagen 500 |
| 6 | 25% citric acid + collagen 500 + 1% Mg |

An osteoblastic cell line (SaOS-2) was used to determine cell viability and proliferation ability.

Cell adhesion and cytotoxicity were assessed using the Resazurin sodium salt (Alamar Blue) assay, and cell morphology was assessed using confocal microscopy (immunofluorescent labelling of F-actin with phalloidin) after 3 days of incubation.

The bacterial strain *S. gordonii* was used to assess bacterial activity. The antibacterial properties of the suggested treatments were determined using the Resazurin sodium salt (Alamar Blue) assay after 1 day of incubation.

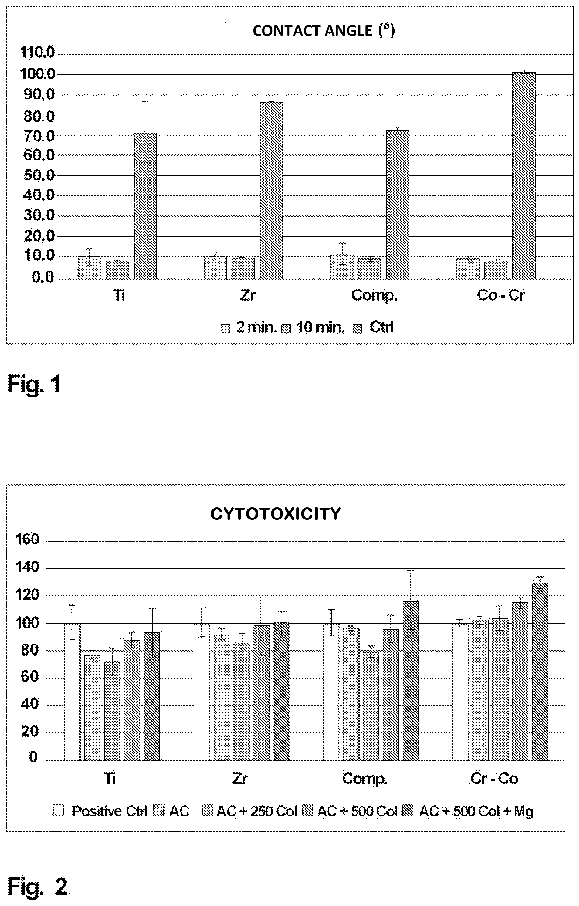

As shown in the graph of Figure 1, treatment of the different materials (titanium, zirconium, composite, and cobalt-chromium) with the solution of the invention significantly reduced the contact angle of the treated samples as compared with the control samples.

The treatments imparted hydrophilic/superhydrophilic properties to the sample surfaces.

The combination of the elements making up the solution of the invention used in this study achieved a cell viability of over 70%, a threshold at which cytotoxicity is considered absent, thereby promoting osteoblast adhesion and proliferation. Furthermore, it imparts antibacterial properties to the surface without damaging the cells.

Figure 2 shows the results of the cytotoxicity assay for the surfaces of the four proposed materials under the five experimental conditions. In all cases, cell viability exceeded 70%, and therefore, treatment with the solution of the invention shows cytocompatibility.

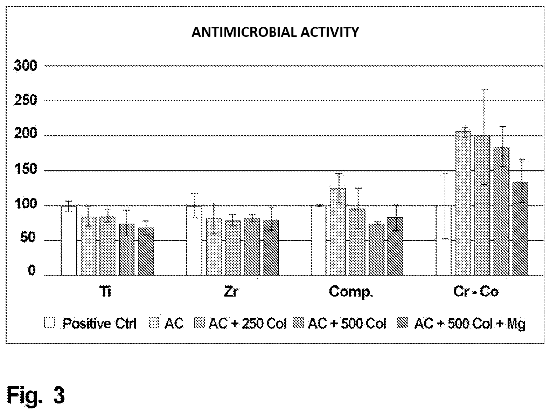

Figure 3 shows the results obtained in the Alamar Blue assay to determine bacterial adhesion and growth on the study surfaces. Titanium, zirconium and composite surfaces showed a favourable response to the four applied treatments, reducing bacterial adhesion on the samples.

On the other hand, none of the applied treatments helped to reduce bacterial growth on the cobalt-chromium surfaces. However, the treatment that stands out due to its results compared with the other treatments is the one performed using the solution of the invention containing 25% citric acid + collagen 500 + 1% Mg.

Having sufficiently described the nature of the invention, it is hereby noted for the relevant purposes that modifications deemed appropriate may be included therein, provided that they do not entail any alteration to the essential characteristics of the invention claimed below.

## Claims

1. A solution for regenerating the passivation layer on dental implants and reducing bacterial activity, **characterised in that** it comprises, in its composition, between 0.2 and 1.0 grams of collagen and between 5 and 50 grams of divalent magnesium or calcium ion salts for every litre of a 10-50% (v/v) citric acid solution.

2. The use of the solution according to claim 1 as a mouthwash for dental health, applied to dental implants and their components.

3. The use of the solution according to claim 1 as an agent for improving the hydrophilicity of the surface of dental implants.

4. The use of the solution according to claim 1 as an agent for improving the osseointegration of dental implants.

5. The use of the solution according to claim 1 as an agent for reducing bacterial activity on the surface of dental implants.
